# EUROPEAN PATENT APPLICATION

(11) **EP 4 477 749 A1**
(43) Date of publication of application: **18.12.2024**
(21) Application number: 23770761.7
(22) Date of filing: 14.03.2023
(51) Int. Cl.: C12N 5/0784, A61K 35/15, A61K 39/00, A61P 35/00, C12N 5/07, C12N 15/12

(54) **METHOD FOR REPROGRAMMING FIBROBLAST OR FIBROBLAST-LIKE CELL TO CONVENTIONAL TYPE-2 DENDRITIC CELL**

(30) Priority: 18.03.2022 JP 2022043701
(71) Applicant: National Institute of Advanced Industrial Science and Technology, Chiyoda-ku Tokyo 100-8921 (JP)
(72) Inventor: KUMAGAI, Yutaro, Tsukuba-shi, Ibaraki 305-8560 (JP)
(74) Representative: HGF
(86) International application number: PCT/JP2023/009757
(87) International publication number: WO 2023/176806

(57) **Abstract**

Provided is a method for producing a conventional type-2 dendritic cell (cDC2)-like cell from a fibroblast or a fibroblast-like cell, the method comprising a step of introducing a nucleic acid encoding PU. 1, a nucleic acid encoding KLF4, a nucleic acid encoding IRF4 and a nucleic acid encoding C/EBP into the fibroblast or the fibroblast-like cell.

## Description

### TECHNICAL FIELD

The present invention relates to a method and to a composition for preparing a conventional type-2 dendritic cell from a fibroblast or a fibroblast-like cell by direct reprogramming.

### BACKGROUND ART

Pancreatic cancer has a 5-year relative survival rate of less than 10% and is known as an intractable cancer with extremely poor prognosis compared to other cancers. One characteristic of intractable cancers, such as pancreatic cancer, is that abundant stroma surrounds the cancer cells. The stroma contains many cells that promote angiogenesis and the proliferation and invasion of cancer cells, and among these, cancer-associated fibroblasts (CAPs) are the main cells in terms of quantity. CAPs not only promote the proliferation of cancer cells by producing growth factors, but also stiffen cancer tissue by overproducing extracellular matrix such as collagen. As a result, intractable cancers are highly resistant to conventional general chemotherapies and radiation therapies.

Immunotherapy to activate an antitumor immune response is being tried as a treatment method for intractable cancer. Of these, the so-called DC vaccine therapy, which activates an anti-tumor immune response by sensitizing autologous dendritic cells with cancer antigens *in vitro* and then returning them to the patient's body, has gained attention as the therapy is also highly safe. However, because the dendritic cells and their precursor cells, monocytes, are present only in very small quantities in peripheral blood and cannot be grown in culture, there are challenges in the preparation of therapeutically effective amounts of DC vaccine. To solve this problem, methods of preparing dendritic cells by reprogramming are currently under development. Techniques in which induced pluripotent stem cells (iPSCs) are prepared from somatic cells and induced into dendritic cells are already being tested in clinical settings. However, as the preparation of iPSCs is time consuming and expensive, as well as technically challenging, the clinical application of iPSC-derived DCs has yet to be realized.

Therefore, there is growing anticipation of direct reprogramming technology which would directly induce differentiated cells into another type of differentiated cell without going through iPSCs. In particular, if dendritic cells could be directly induced from fibroblasts, DC vaccines could be prepared easily, quickly, and at low cost. It has now been demonstrated that mouse embryonic fibroblasts (MEFs) can be directly reprogrammed to dendritic cells (Patent Documents 1 and 2, and Non-Patent Document 1). However, it has not yet been possible to directly induce adult human fibroblasts into dendritic cells with high efficiency.

### REFERENCE DOCUMENT LIST

### PATENT DOCUMENTS

| | |
|---|---|
| Patent Document 1: | WO 2018/185709 |
| Patent Document 2: | WO 2021/105234 |

### NON-PATENT DOCUMENTS

Non-Patent Document 1: Rosa FF et al., Sci. Immunol., 2018 Dec 7;3(30):eaau4292

### SUMMARY OF INVENTION

### PROBLEM TO BE SOLVED BY THE INVENTION

An object of the present invention is to provide a novel therapeutic strategy for intractable cancer and an easy and efficient method for preparing a DC vaccine.

### MEANS FOR SOLVING THE PROBLEMS

The present inventors have intensively researched, and as a result, succeeded in transdifferentiating various fibroblasts or fibroblast-like cells into conventional type-2 dendritic cell (cDC2)-like cells by introducing a combination of four specific transcription factors.

That is, according to one embodiment, the present invention provides a method for producing a conventional type-2 dendritic cell (cDC2)-like cell from a fibroblast or a fibroblast-like cell, the method comprising a step of introducing a nucleic acid encoding PU.1, a nucleic acid encoding KLF4, a nucleic acid encoding IRF4 and a nucleic acid encoding C/EBP, into the fibroblast or the fibroblast-like cell.

The C/EBP is preferably C/EBP alpha or C/EBP beta.

The fibroblast or fibroblast-like cell is preferably a cancer-associated fibroblast.

The fibroblast or fibroblast-like cell is preferably a mesenchymal stem cell.

In addition, according to one embodiment, the present invention provides a pharmaceutical composition for preventing or treating cancer in a subject, the pharmaceutical composition comprising conventional type-2 dendritic cell (cDC2)-like cells prepared by the above method.

The cDC2-like cell is preferably loaded with a cancer antigen.

The cDC2-like cell is preferably autologous to the subject.

The cDC2-like cell may be immortalized.

In addition, according to one embodiment, the present invention provides a composition for transdifferentiating a fibroblast or a fibroblast-like cell into a conventional type-2 dendritic cell-like cell, the composition comprising a nucleic acid encoding PU. 1, a nucleic acid encoding KLF4, a nucleic acid encoding IRF4 and a nucleic acid encoding C/EBP.

### EFFECTS OF INVENTION

According to the method of the present invention, a fibroblast or fibroblast-like cell can be directly transdifferentiated into a cDC2-like cell. Thus, according to the method of the present invention, a DC vaccine can be prepared easily and efficiently.

### BRIEF DESCRIPTION OF DRAWINGS

FIG. 1 is a figure showing the results of flow cytometry gating MEFs introduced with four mouse transcription factors, based on the expression of CD45, CD11b, CD11c and MHC class II.
FIG. 2 is a figure showing the expression of cDC2-specific markers (CLEC10A, SIRPα and CCR7) in CD45+CD11b+MHC-II+CD11c+ cells.
FIG. 3 is a figure showing the expression of C-type lectins (SIGNR1 and DC-SIGN) in CD45+CD11b+MHC-II+CD11c+ cells.
FIG. 4 is a figure showing the expression of macrophage-specific markers (CD115 and F4/80) and a monocyte-specific marker (Ly6C) in CD45+CD11b+MHC-II+CD11c+ cells.
FIG. 5 is a graph showing the results of quantifying the expression of the cDC-specific transcription factor Zbtb46 in icDC2 by RT-qPCR.
FIG. 6 is a graph showing the results of quantifying the expression of the macrophage-specific tyrosine kinase MerTK in icDC2 by RT-qPCR.
FIG. 7 is a graph showing the time course of the transdifferentiation of immortalized MEFs into which four mouse transcription factors have been introduced, into cDC2-like cells.
FIG. 8 is a graph showing the proliferation of cDC2-like cells induced from immortalized MEFs.
FIG. 9 is a graph showing the transdifferentiation efficiency of MEFs into which mouse PU. 1 in combination with one or more transcription factors selected from mouse KLF4, IRF4 and C/EBP alpha have been introduced, into icDC2.
FIG. 10 is a schematic diagram showing the structure of a polycistronic vector expressing four mouse transcription factors under the control of a TRE promoter.
FIG. 11 is a figure showing the results of flow cytometry gating MEFs into which the four mouse transcription factors have been introduced by individual vectors, based on the expression of CD45, CD 11b, CD11c and MHC class II.
FIG. 12 is a figure showing the results of flow cytometry gating MEFs into which the four transcription factors have been introduced by a single polycistronic vector, based on the expression of CD45, CD 11b, CD11c and MHC class II.
FIG. 13 is a figure showing the results of flow cytometry gating MRC-5s into which the four human transcription factors, based on the expression of CD45 and CD11b.
FIG. 14 is a figure showing the increased expression of HLA-DR in CD45+CD11b+ cells.
FIG. 15 is a figure showing the increased expression of CD11c in CD45+CD11b+ cells.
FIG. 16 is a plot showing the increase in the number of CD3 epsilon-positive cells in a mixed cell population of MEFs, MSCs, or 3T3s, into which the four mouse transcription factors have been introduced, and CD4-positive T cells.
FIG. 17 is a figure showing the increased expression of CD69 in CD4-positive T cells mixed with MEFs into which the four mouse transcription factors have been introduced.
FIG. 18 is a graph comparing the tumor size in model mice which are administered or are not administered with MEFs into which the four mouse transcription factors have been introduced.

### MODE FOR CARRYING OUT THE INVENTION

Hereinafter, the present invention will be described in detail, but the present invention is not limited to the embodiments described in the present specification.

According to the first embodiment, the present invention is a method for producing a conventional type-2 dendritic cell (cDC2)-like cell from a fibroblast or a fibroblast-like cell, the method comprising a step of introducing a nucleic acid encoding PU. 1, a nucleic acid encoding KLF4, a nucleic acid encoding IRF4 and a nucleic acid encoding C/EBP into the fibroblast or the fibroblast-like cell.

"Fibroblast" refers to a spindle-shaped cell present in the stroma, which produces extracellular matrix proteins and degrading enzymes therefor. On the other hand, a "fibroblast-like cell" refers to a cell which is defined solely by the spindle shape similar to that of a fibroblast, but the functions of which, such as the ability to differentiate, are not specified. Morphologically, a fibroblast and a fibroblast-like cell cannot be distinguished. In the method of the present embodiment, either a fibroblast or a fibroblast-like cell can be used.

The fibroblast or fibroblast-like cell according to the present embodiment may be derived from any vertebrate, but is preferably derived from a mammal such as a mouse, rat, guinea pig, rabbit, dog, sheep, pig, cow, horse, goat, monkey, or human, and is particularly preferably derived from a human. In addition, the fibroblast or fibroblast-like cell according to the present embodiment may be derived from any embryonic or adult tissue, and the tissue may be either normal or cancerous tissue.

Accordingly, examples of the fibroblast or fibroblast-like cell according to the present embodiment include, but are not limited to, a mouse embryonic fibroblast (MEF), an immortalized mouse embryonic fibroblast (3T3), an adult mouse tail tip fibroblast (TTF), a human embryonic fibroblast (HEF), a cancer-associated fibroblast (CAF), an adipose-derived mesenchymal stem cell (AD-MSC), and a bone marrow mesenchymal stem cell (BM-MSC).

The methods for preparing fibroblasts or fibroblast-like cells have been sufficiently established, and the fibroblast or fibroblast-like cell can be prepared according to methods known in the art. Alternatively, an already established fibroblast line or fibroblast-like cell line may be obtained, for example, from the Riken BioResource Center (Riken BRC) or the ATCC (American Type Culture Collection).

In the method of the present embodiment, nucleic acids encoding PU.1, KLF4, IRF4 and C/EBP are introduced into a fibroblast or a fibroblast-like cell.

"PU.1", "KLF4", "IRF4" and "C/EBP" are all transcription factors. The C/EBP family consists of C/EBP alpha, C/EBP beta, C/EBP gamma, C/EBP delta, C/EBP epsilon and CHOP (C/EBP zeta). The C/EBP according to the present embodiment may be any of these, but is preferably C/EBP alpha or C/EBP beta. PU.1, KLF4, IRF4 and C/EBP according to the present embodiment may be derived from any vertebrate, but are preferably derived from a mammal such as a mouse, rat, guinea pig, rabbit, dog, sheep, pig, cow, horse, goat, monkey, or human, and is particularly preferably derived from a human.

The genes encoding PU.1, KLF4, IRF4 and C/EBP have already been cloned, and their nucleic acid sequence information can be obtained from a given database. For example, NM_003120 can be used for human PU.1, NM_011355 for mouse PU.1, NM_004235 for human KLF4, NM_010637 for mouse KLF4, NM_002460 for human IRF4, NM_013674 for mouse IRF4, NM_004364 for human C/EBP alpha, NM_007678 for mouse C/EBP alpha, NM_005194 for human C/EBP beta, and NM_009883 for mouse C/EBP beta (all from the NCBI Nucleotide Database).

PU. 1, KLF4, IRF4 and C/EBP according to the present embodiment may also include the variants and homologues thereof having equivalent transcriptional regulatory activity. In other words, PU. 1, KLF4, IRF4, and C/EBP according to the present embodiment can include proteins composed of amino acid sequences having 80% or more, preferably 90% or more, and more preferably about 95% or more identity with the amino acid sequences registered in the database, as long as their transcriptional regulatory activity is maintained. Amino acid sequence identity can be calculated using a sequence analysis software or a program conventionally used in the art (FASTA, BLAST, etc.). In addition, PU.1, KLF4, IRF4, and C/EBP according to the present embodiment can include proteins consisting of amino acid sequences registered in the database in which one to several amino acids have been substituted, deleted, inserted, and/or added, as long as their transcriptional regulatory activity is maintained. Here, "one to several" is, for example, "1 to 30", preferably "1 to 10", and particularly preferably "1 to 5".

The nucleic acids encoding PU. 1, KLF4, IRF4 and C/EBP can be introduced into a fibroblast or a fibroblast-like cell by methods well known in the art. For example, these nucleic acids can be cloned into an expression vector and introduced into a cell. Regarding the type of expression vector, a suitable expression vector can be selected according to the target fibroblast or fibroblast-like cell. For example, it may be, but is not limited to, viral vectors such as retrovirus, lentivirus, adenovirus, and Sendai virus, and plasmid vectors such as pCMV can be used. In addition, the nucleic acids encoding PU.1, KLF4, IRF4 and C/EBP may be each expressed from individual vectors, or expressed from a single polycistronic vector.

In the method of the present embodiment, the expression vector can be introduced into a cell by a method well known in the art, depending on the type of vector. If a non-viral vector is used, it can be introduced, for example, by lipofection, electroporation, or microinjection. If a viral vector is used, it can be introduced by infecting a cell at the appropriate titer or multiplicity of infection (MOI).

When PU.1, KLF4, IRF4 and C/EBP are expressed, the fibroblast or fibroblast-like cell is transdifferentiated into a conventional type-2 dendritic cell (cDC2)-like cell. Here, "transdifferentiation (or direct reprogramming)" refers to the direct conversion of one mature (differentiated) cell type to another mature (differentiated) cell type without passing through a pluripotent cell state.

In the present embodiment, a "conventional type-2 dendritic cell (cDC2)-like cell" refers to a cell expressing cDC2-specific markers and having antigen-presenting ability. Dendritic cells (DCs) are classified into three major subsets: conventional type-1 dendritic cells (cDC1s), conventional type-2 dendritic cells (cDC2s), and plasmacytoid dendritic cells (pDCs), and the markers that characterize each subset are well known. The cDC2-like cell according to the present embodiment may be defined by any combination of well-known blood cell markers, myeloid cell markers, and cDC2-specific markers, but are preferably defined, for example, as CD45+, CD11b+, CD11c+, and MHC class II (HLA-DR)+. A significant expression of MHC class II (HLA-DR) can indicate that the cDC2-like cell of the present embodiment is capable of antigen presentation. In addition to the above markers, the cDC2-like cell according to the present embodiment preferably further expresses CLEC10A, SIRPα, and/or CCR7, and more preferably further expresses a C-type lectin such as SIGNR1 and DC-SIGN. The cDC2-like cell according to the present embodiment may further express not only a cDC2-specific marker, but also a macrophage-specific marker such as CD115 and a monocyte-specific marker such as Ly6C. The marker expression can be analyzed by a known method such as RT-PCR, Western blotting, and flow cytometry.

According to the method of the present embodiment, a fibroblast or fibroblast-like cell can directly produce a cDC2-like cell without passing through a pluripotent cell state. Since fibroblasts can be easily obtained and proliferated, the method of the present embodiment is useful in the preparation of DC vaccines.

According to the second embodiment, the present invention is a pharmaceutical composition for preventing or treating cancer in a subject, the pharmaceutical composition comprising cDC2-like cells prepared by the above method.

In the present embodiment, "preventing" includes not only preventing the onset of cancer in a subject at risk of developing cancer, but also reducing the risk of developing cancer, or delaying the progression or reducing the severity of symptoms when a subject has developed cancer by treating the subject before the onset of cancer. The term "treating" includes not only the complete cure of a cancer, but also remission or alleviation of cancer symptoms, delay or arrest of the cancer progression, and improvement of the cancer prognosis.

The "subject" according to the present embodiment may be any vertebrate, but is preferably a mammal such as a mouse, rat, rabbit, sheep, pig, cow, goat, monkey, or human, and is particularly preferably a human. The subject can be of any age and may be an infant, child, adolescent, adult, and elderly subjects.

The cDC2-like cell according to the present embodiment is the same as that defined in the first embodiment, and may be prepared from a fibroblast or fibroblast-like cell derived from any tissue of any vertebrate. The cDC2-like cell according to the present embodiment can be prepared from a fibroblast or fibroblast-like cell which is preferably allogeneic or autologous, and is more preferably autologous to the subject.

The cDC2-like cell according to the present embodiment may optionally be immortalized. In this context, an "immortalized" cell means that the cell remains capable of proliferating even after a repeated number of divisions, i.e., the cell has infinite proliferative capacity. Methods for immortalizing cells have already been established, and a known method can be employed. For example, a cell can be immortalized by introducing an immortalizing gene, such as the SV40T antigen gene and telomerase reverse transcriptase (TERT) gene, into the cell by a retroviral vector.

The cDC2-like cell according to the present embodiment is preferably loaded with a cancer antigen. The methods for loading cancer antigens on dendritic cells have been sufficiently established, and the cDC2-like cell according to the present embodiment can be loaded with a cancer antigen according to a method known in the art. For example, cDC2-like cells may be cultured for 12 hours to several days in a medium containing full-length cancer antigen peptides or partial fragments thereof or tumor lysates. Alternatively, a nucleic acid encoding a full-length cancer antigen peptide or a partial fragment thereof may be introduced into a cell using a viral or non-viral vector.

The cancer antigen that can be used in the present embodiment is not particularly limited, and it may be, for example, HER2/NEU, TERT, WT1, MAGE-A3, NY-ESO-1, PAP, PSA, or the like.

The pharmaceutical composition of the present embodiment contains the above cDC2-like cell as an active ingredient. The pharmaceutical composition of the present embodiment may be composed of only the active ingredient, but it may further contain, as optional components, known pharmaceutically acceptable carriers, buffers, other components (e.g., Toll-like receptor ligands such as CpG DNA, STING agonists such as synthetic cyclic nucleotides, and cytokines/chemokines such as GM-CSF, CCL19, and FLT3L), and the like. For example, the pharmaceutical composition of the present embodiment can be prepared as an injectable form using a carrier such as phosphate-buffered saline that can maintain the survival of the cells that are the active ingredient. In this case, the cDC2-like cells may be suspended in the above carrier at a concentration of, for example, 1×10⁶ to 1×10⁸ cells/mL.

The pharmaceutical composition of the present embodiment can be administered by an appropriate method such as by injection, infusion, or transplant. Preferably, the pharmaceutical composition of the present embodiment may be injected intravenously, subcutaneously, intradermally, or into a lymph node. The dosage of the pharmaceutical composition of the present embodiment may vary depending on age, weight, and cancer severity of a subject, and it may be, for example, 1×10⁵ to 1×10¹⁰ cells/kg (body weight). The dose may be administered once or multiple times.

The pharmaceutical composition of the present embodiment can safely and effectively activate an anti-tumor immune response. Therefore, it is useful in the treatment of intractable cancer that has been difficult to treat with conventional chemotherapy and radiation therapy.

According to the third embodiment, the present invention is a composition for transdifferentiating a fibroblast or a fibroblast-like cell into a conventional type-2 dendritic cell-like cell, the composition comprising a nucleic acid encoding PU. 1, a nucleic acid encoding KLF4, a nucleic acid encoding IRF4 and a nucleic acid encoding C/EBP. The cDC2-like cell, fibroblast, fibroblast-like cell, and the nucleic acids encoding PU. 1, KLF4, IRF4 and C/EBP according to the present embodiment are the same as those defined in the first embodiment, and can be prepared and used as described in the first embodiment.

### EXAMPLES

Hereinafter, the present invention will be further described with reference to Examples. However, the present invention is not in any way limited thereto.

### 1. Transdifferentiation of MEFs into dendritic cells by four transcription factors

The coding sequence of the mouse PU.1 gene (RefSeq ID: NM_011355, SEQ ID No: 1), the coding sequence of the KLF4 gene (RefSeq ID: NM_010637, SEQ ID No: 2), the coding sequence of the IRF4 gene (RefSeq ID: NM_013674, SEQ ID No: 3) and the coding sequence of the C/EBP alpha gene (RefSeq ID: NM_007678, SEQ ID No: 4) were amplified by PCR and subcloned into pMXs vectors (Kitamura et al., Exp. Hematol., 2003). These vectors were transfected into HEK293 cells along with a VSV-G plasmid and a gag/pol plasmid (Addgene) to produce retroviral particles. The culture supernatant was collected, and ultracentrifugation was performed at 4°C, 6,000×g for 20 minutes. The supernatant from which the insoluble fraction was removed was mixed with 1/4 volume of 40% (w/v) PEG-8000, 1.2 M NaCl/PBS solution and incubated overnight at 4°C. Then, ultracentrifugation was performed at 4°C, 1,600×g for 60 minutes to remove the supernatant. Pellets were suspended by adding 50 µL of DMEM per 1 mL of the original culture supernatant and stored at -80°C.

Mouse embryonic fibroblasts (MEFs) were obtained from 13.5-day-old embryos according to a conventional method. The MEFs were placed in a 24-well plate (4×10⁴ cells per well) and 100 µL of virus suspension per well was added on the following day. The following day (designated as day 1), the cells in one well were subcultured in three wells and maintained until measurement. DMEM containing 10% FCS was used for culture. After 13 days, the cells were collected and immunostained for marker proteins. To stain the marker proteins, the cells collected by trypsinization were suspended in an antibody solution and incubated at 37°C for 20 minutes, then at 4°C for 15 minutes. After staining, the cells were observed by FACS Verse (BD Biosciences).

**Table 1. Antibodies used to stain markers**

| [Table 1] | | | |
|---|---|---|---|
| Antigen | Species | Clone | Source |
| CD45 | Mouse | 30-F11 | Tonbo Biosciences |
| CD11b | Mouse | M1/70 | BD Pharmingen |
| MHC-II | Mouse | M5/114.15.2 | Tonbo Biosciences |
| CD11c | Mouse | N418 | BD Pharmingen |
| CD80 | Mouse | 16-10A1 | Biolegend |
| CD86 | Mouse | GL1 | Tonbo Biosciences |
| CD3epsilon | Mouse | 145-2C11 | Biolegend |
| CD44 | Mouse | IM7 | BD Pharmingen |
| CLEC10A | Mouse | 7H11 | Biolegend |
| CD69 | Mouse | H1.2F3 | Biolegend |
| SIRPalpha | Mouse | P84 | Biolegend |
| CCR7 | Mouse | 4B12 | Biolegend |
| DC-SIGN | Mouse | LWC06 | eBioscience |
| SIGNR1 | Mouse | 22D1 | eBioscience |
| CD115 | Mouse | AFS98 | Biolegend |
| F4/80 | Mouse | BM8 | Biolegend |
| Ly6C | Mouse | AL-21 | BD Pharmingen |
| CD45 | Human | HI30 | Tonbo Biosciences |
| CD11b | Human | ICRF44 | Biolegend |
| HLA-DR | Human | L243 | Tonbo Biosciences |
| CD11c | Human | Bu15 | Biolegend |

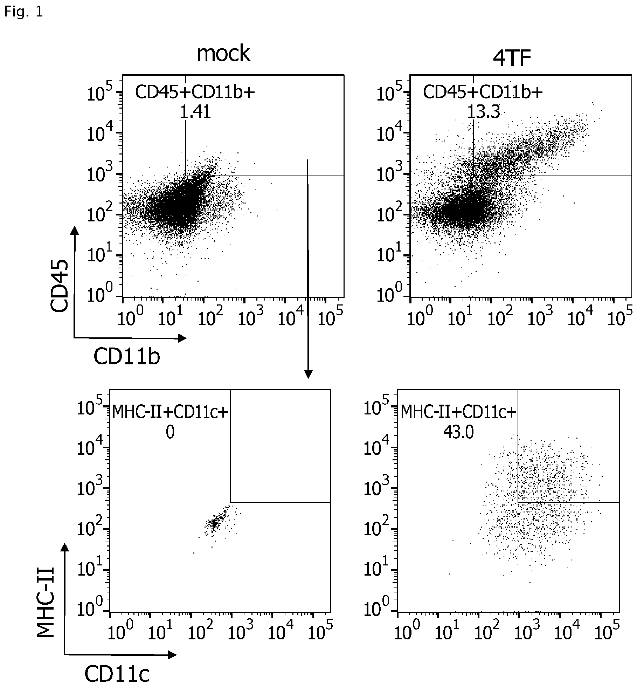

The results of flow cytometry gating the cells based on the expression of the blood cell marker CD45, myeloid cell marker CD11b, dendritic cell marker CD11c and antigen-presenting cell marker MHC class II (MHC-II) are shown in FIG. 1. In the figure, "4TF" indicates the cells introduced with the four transcription factors PU. 1, KLF4, IRF4 and C/EBP alpha, and "mock" indicates cells without introduction treatment. The results confirmed that dendritic cell-like cells expressing the blood cell marker CD45, myeloid cell marker CD11b, dendritic cell marker CD11c and antigen-presenting cell marker MHC class II were obtained from the cells introduced with the four transcription factors.

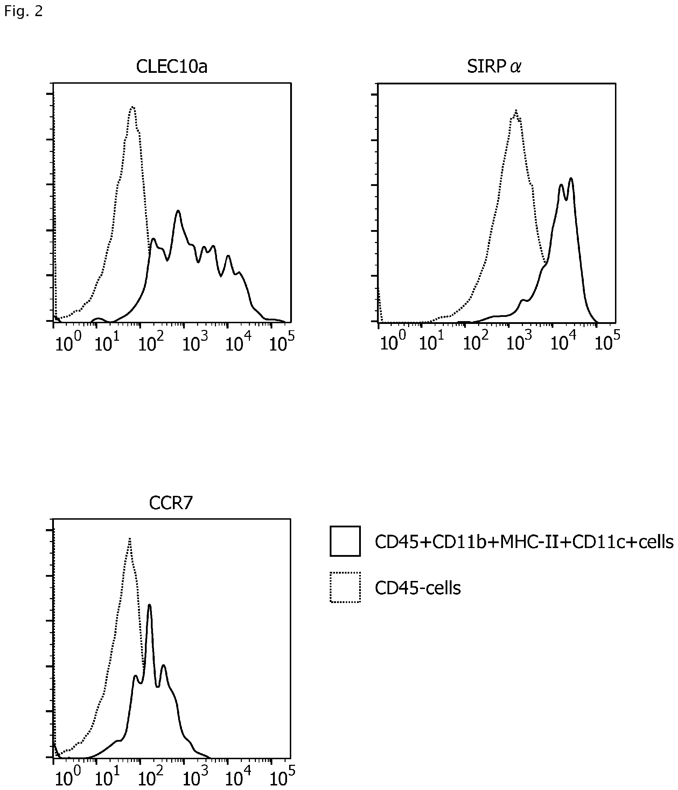

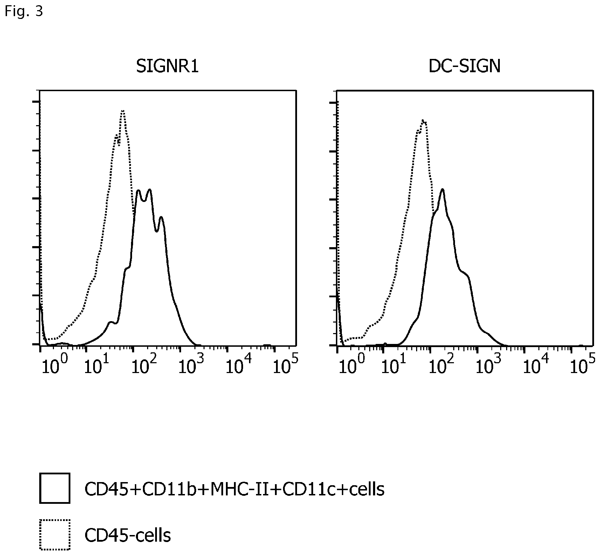

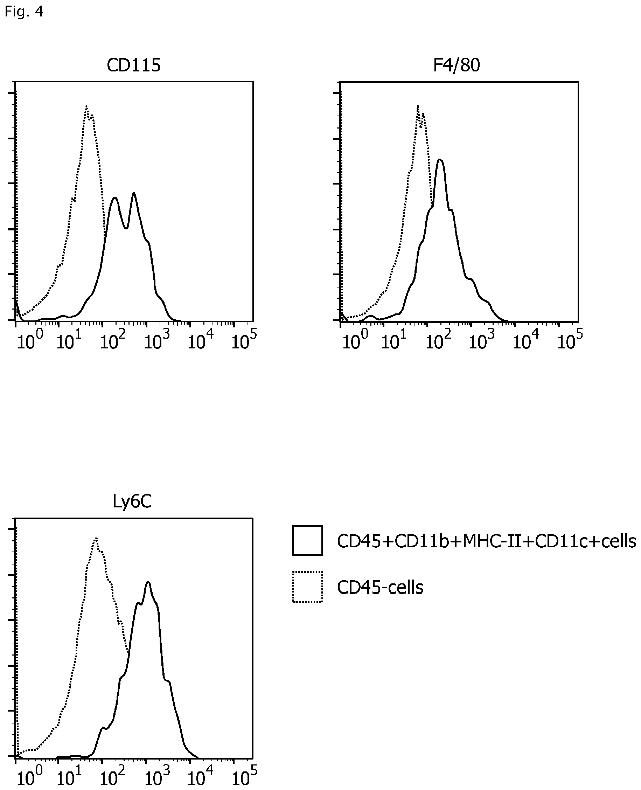

The results of comparing the expression of cDC2-specific markers and other monocytic cell markers between CD45+CD11b+MHC-II+CD11c+ cells and CD45- cells are shown in FIGS. 2 to 4. The CD45+CD11b+MHC-II+CD11c+ cells were found to express the cDC2-specific markers CLEC10A, SIRPα and CCR7 (FIG. 2). The CD45+CD11b+MHC-II+CD11c+ cells also expressed the C-type lectins SIGNR1 and DC-SIGN which are expressed in dendritic cells (FIG. 3). On the other hand, the CD45+CD11b+MHC-II+CD11c+ cells differed from cDC2 in expressing macrophage-specific markers (CD115 and F4/80) and the monocyte-specific marker Ly6C. Based on these results, the cells obtained by introducing the four transcription factors PU. 1, KLF4, IRF4 and C/EBP alpha were concluded to be cDC2-like cells, and were named "induced cDC2 (icDC2)".

Next, the expression of the cDC-specific transcription factor Zbtb46 and the macrophage-specific tyrosine kinase MerTK in icDC2 was evaluated by RT-qPCR. GM-CSF-induced bone marrow-derived dendritic cells (GM-DCs) were prepared by the following procedure and used as positive controls. Bone marrow cells were harvested from the femur of mice, and bone marrow cells for one animal were suspended in 24 mL of GM-CSF medium (RPMI 1640 medium containing 10 ng/mL GM-CSF (Peprotech), 3.5 µL of 2-mercaptoethanol (Nacalai Tesque), and 10% FCS), then placed in a 24-well plate at 1 mL per well. Half of the culture supernatant was replaced by new medium every 2 days. Six days after the start of culture, the floating cells were collected and used as GM-DCs.

RNA was extracted from the cells using TRI reagent (Molecular Research Center) according to the instructions, and cDNA was synthesized using ReverTra Ace qPCR RT Master Mix with gDNA Remover (Toyobo) according to the instructions. Using this cDNA as a template, qPCR was performed using THUNDERBIRD SYBR qPCR Mix (Toyobo) and LightCycler 96 (Roche). The primers used are shown below. The fold change in induction of expression was calculated using the expression level in the mock cells as 1.

**Table 2. Primers used for qPCR**

| [Table 2] | | |
|---|---|---|
| Primer name | Sequence (5'→3') | SEQ ID No. |
| Zbtb46_Fw | cagcagccgagactcaaatg | 9 |
| Zbtb46_Rv | gtaggccctcatcgatgtga | 10 |
| Mertk_Fw | atggaaaggaattgctcggg | 11 |
| Mertk_Rv | cacactggctatgctgaaca | 12 |

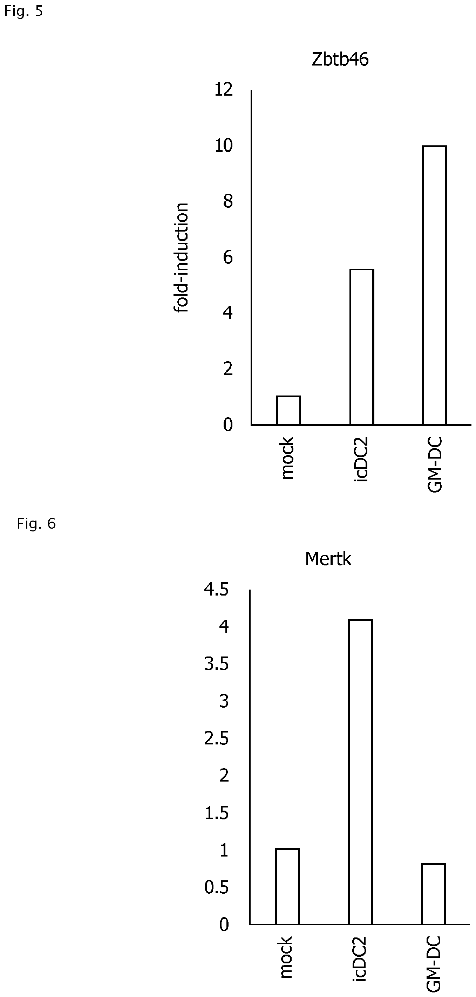

The results are shown in FIGS. 5 and 6. The expression of the Zbtb46 gene and the Mertk gene was increased in icDC2. These results indicate that icDC2 is a novel cDC2-like cell that has characteristics of cDC2, but also has different characteristics from cDC2.

### 2. Time-series of transdifferentiation of MEFs into cDC2-like cells by four transcription factors

MEFs immortalized by transfection with SV40T antigen-expressing plasmid, pBSSVD2005 (Addgene, plasmid #21826), were introduced with four transcription factors by the same procedure as disclosed in 1 above, and the expression of the markers was observed. The time course of the transdifferentiation was analyzed based on the proportion of CD45+CD11b+MHC-II+CD11c+ cell fractions.

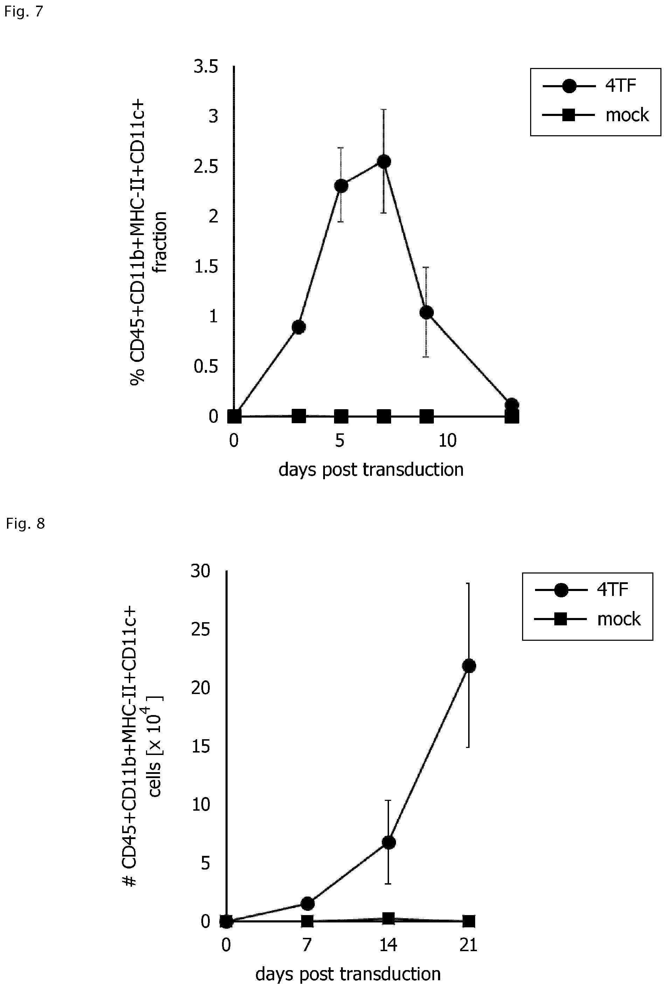

The results are shown in FIG. 7. Transdifferentiation was observed to begin around three days after the introduction of the four transcription factors, peaking at 7 days, and declining thereafter.

### 3. Proliferative capacity of immortalized MEF-derived icDC2

Next, the cells of 2 above were subcultured every 7 days and the number of CD45+CD11b+MHC-II+CD11c+ cells was measured. The results are shown in FIG. 8. The number of CD45+CD11b+MHC-II+CD11c+ cells increased as the number of days of culture increased, indicating that these cells themselves were proliferating. These results indicate that immortalized cell-derived icDC2 can be maintained in proliferation, which indicates a potential for application to drug discovery.

### 4. Combination of transcription factors required for transdifferentiation of MEFs into cDC2-like cells

It was examined which of PU.1, KLF4, IRF4, and C/EBP alpha are required for the transdifferentiation of MEFs into cDC2-like cells. Because CD45-positive cells were not induced by any combination of transcription factors without PU.1 (data not shown), PU. 1 in combination with one or more transcription factors selected from KLF4, IRF4 and C/EBP alpha were introduced into MEFs, and the efficiency of transdifferentiation into icDC2 was evaluated by the same procedure as disclosed in 1 above.

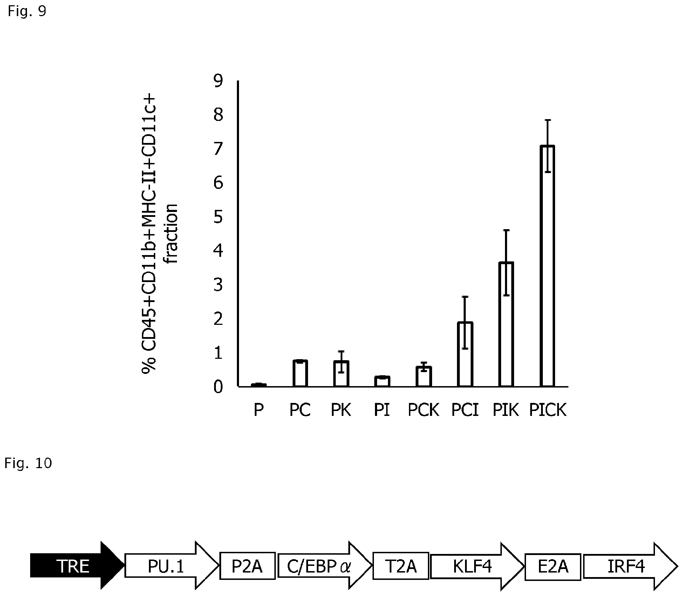

The results are shown in FIG. 9. In the figure, "P" indicates the introduction of PU.1 only; "PC" indicates PU.1 and C/EBP alpha; "PK" indicates PU.1 and KLF4; "PI" indicates PU.1 and IRF4; "PCK" indicates PU.1, C/EBP alpha and KLF4; "PCI" indicates PU.1, C/EBP alpha and IRF4; "PIK" indicates PU. 1, IRF4 and KLF4; and "PICK" indicates the combination of PU.1, IRF4, C/EBP alpha and KLF4. Little differentiation conversion to icDC2 was induced without IRF4. Without KLF4 or C/EBP alpha, transdifferentiation into icDC2 was induced, but the efficiency was significantly lower compared to when all four transcription factors were introduced. These results indicate that the introduction of the combination of all four transcription factors, PU. 1, KLF4, IRF4, and C/EBP alpha, was required for the transdifferentiation of MEFs into cDC2-like cells.

Next, a comparison was made for the transdifferentiation efficiency by other C/EBP families: C/EBP beta (RefSeq ID: NM_009883, SEQ ID No: 13), C/EBP delta (RefSeq ID: NM_007679, SEQ ID No: 14) and C/EBP epsilon (RefSeq ID: NM_207131, SEQ ID No: 15). By the same procedure as disclosed in 1 above, retroviral vectors expressing each C/EBP family were prepared and introduced into MEFs in combination with PU. 1, KLF4 and IRF4 to evaluate the transdifferentiation efficiency. As a result, C/EBP beta exhibited a comparable transdifferentiation efficiency to C/EBP alpha, whereas C/EBP delta or C/EBP epsilon had reduced transdifferentiation efficiency (data not shown). These results lead to the conclusion that the combination of PU. 1, KLF4, IRF4, and C/EBP alpha or C/EBP beta was optimal for the transdifferentiation of MEFs into cDC2-like cells.

### 5. Transdifferentiation of MEFs into cDC2-like cells by polycistronic expression of four transcription factors from a lentiviral vector

A fusion gene in which the four transcription factors PU. 1, KLF4, IRF4, and C/EBP alpha, are connected by the 2A peptides was subcloned into the FUW-tetO-MCS vector (Hockemeyer et al., Cell Stem Cell, 2008) to produce a vector construct that polycistronically expresses the four transcription factors under the control of a tetracycline response element (TRE) promoter (FIG. 10). The produced vector was introduced into HEK293 cells along with pMD2.G (Addgene, plasmid #12259) and psPAX2 (Addgene, plasmid #12260) to produce lentiviral particles. A virus concentrate was prepared by the same procedure as disclosed in 1 above and stored at -80°C. Lentiviral particles for each transcription factor were also produced by the same procedure using the genes of PU.1, KLF4, IRF4 or C/EBP alpha instead of the above fusion gene. The MEFs were placed in a 24-well plate (4×10⁴ cells per well) and 100 µL of virus suspension per well was added on the following day. The following day, 1 µg/mL doxycycline (Sigma-Aldrich) was added, and the medium was changed every other day. After 13 days, the expression of the markers was observed by the same procedure as disclosed in 1 above.

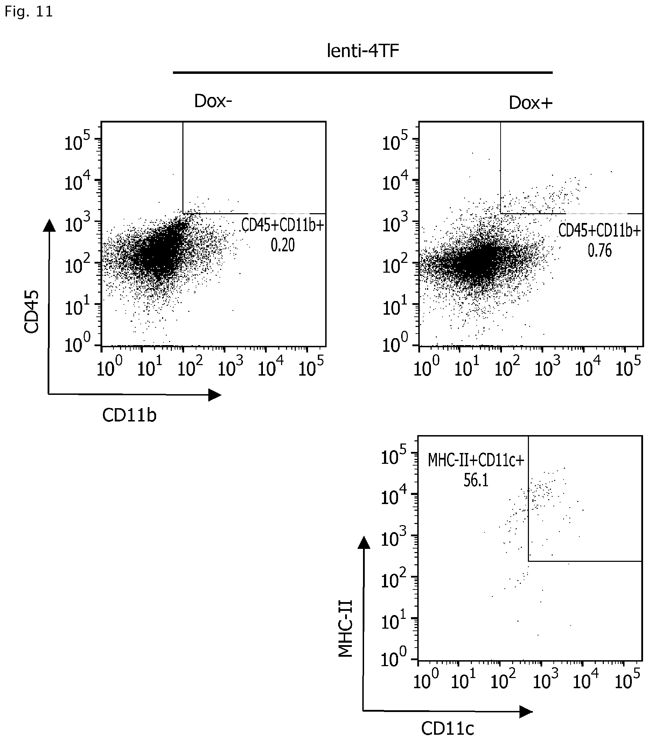

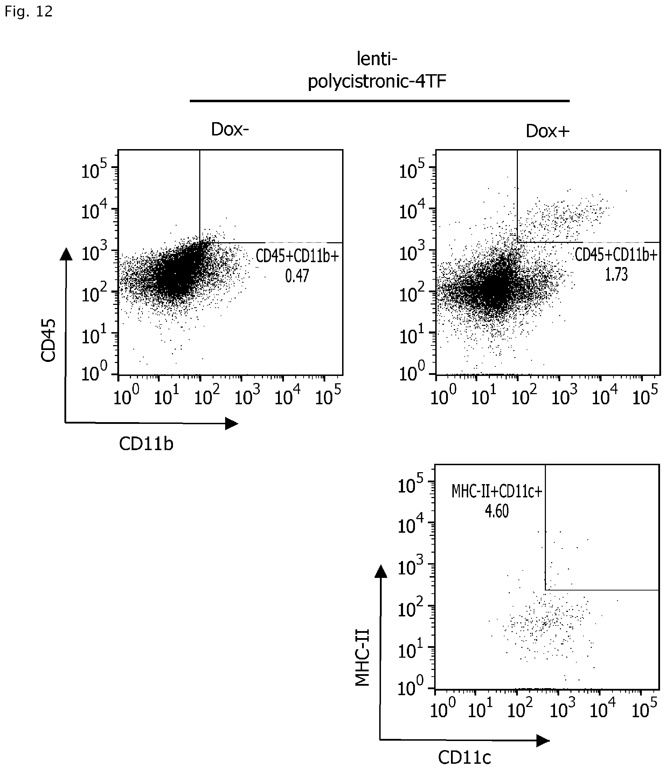

The results of introducing the four transcription factors by individual vectors are shown in FIG. 11, and the results of introducing them by a polycistronic vector are shown in FIG. 12. In both cases, it was confirmed that icDC2 was obtained by the addition of doxycycline (Dox+).

### 6. Study of starting cells to be transdifferentiated into cDC2-like cells by four transcription factors

To examine the types of starting cells that can be transdifferentiated into cDC2-like cells by the introduction of the four transcription factors, the mouse melanoma cell line B16F1 (Riken), the mouse lung cancer cell line 3LL (National Institute of Biomedical Innovation), the mouse breast cancer cell line e0771 (CH3 BioSystems), the mouse lymphoma cell line EL4 (Riken), adult mouse tail tip fibroblasts (TTF) and mouse adipose-derived mesenchymal stem cells (AD-MSC) were used instead of MEFs, and were analyzed in the same manner as disclosed in 1 above. The TTFs were prepared from C57BL/6 mice by a conventional method (Takahashi et al., Nat. Protocol, 2007). The AD-MSCs were prepared by digesting white adipose tissue isolated from C57BL/6 mice with a collagenase solution (RPMI 1640 medium containing 100 U/mL collagenase (Wako) and 5% FCS) and obtaining adhering cells therefrom. All cells were cultured in DMEM containing 10% FCS.

As a result, TTFs and AD-MSCs were transdifferentiated into cDC2-like cells, while B16F1, 3LL, e0771 and EL4 did not transdifferentiate into cDC2-like cells (data not shown). These results indicate that fibroblasts and fibroblast-like cells can be used to produce cDC2-like cells by introduction of the four transcription factors.

### 7. Transdifferentiation of human fibroblasts into cDC2-like cells by human transcription factors

Instead of the coding sequence of the mouse PU.1 gene (SEQ ID No: 1), the coding sequence of the KLF4 gene (SEQ ID No: 2), the coding sequence of the IRF4 gene (SEQ ID No: 3) and the coding sequence of the C/EBP alpha gene (SEQ ID No: 4), the coding sequence of the human PU.1 gene (RefSeq ID: NM_003120, SEQ ID No: 5), the coding sequence of the KLF4 gene (RefSeq ID: NM_004235, SEQ ID No: 6), the coding sequence of the IRF4 gene (RefSeq ID: NM_002460, SEQ ID No: 7) and the coding sequence of the C/EBP alpha gene (RefSeq ID: NM_004364, SEQ ID No: 8) were used to prepare retroviruses by the same procedure as disclosed in 1 above. Using the human fibroblast cell line MRC-5 (ATCC) instead of MEFs, the expression of the markers was observed 13 days after infection by the same procedure as disclosed in 1 above.

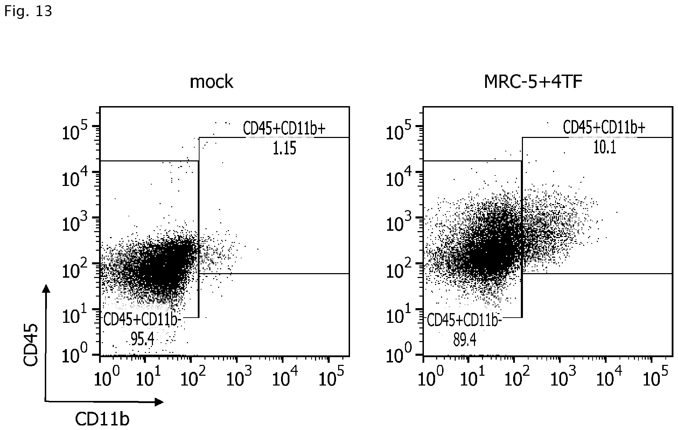

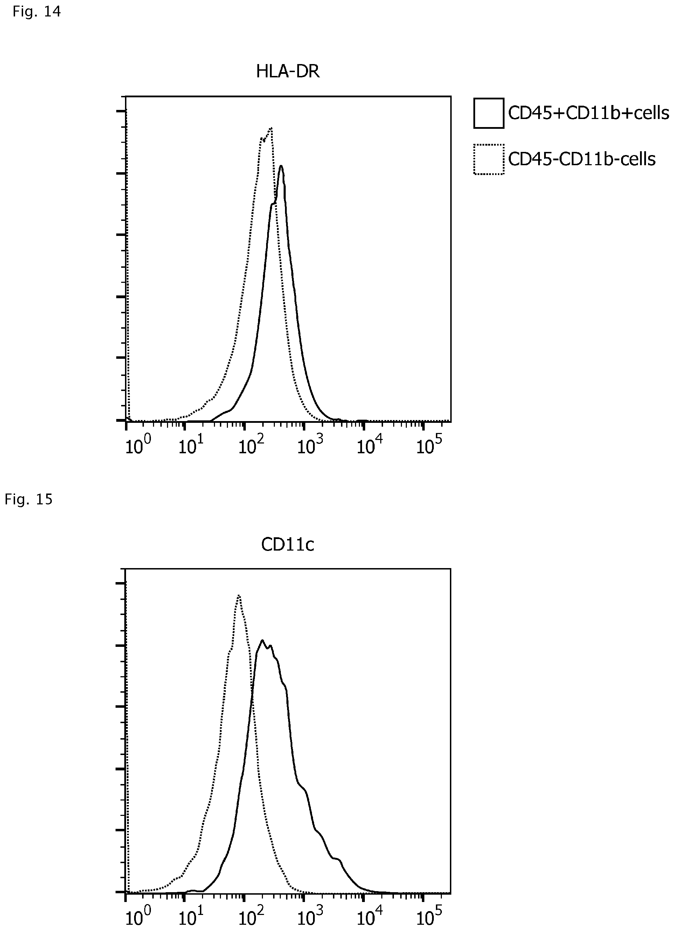

The results are shown in FIGS. 13 to 15. Introduction of the four transcription factors resulted in CD45+CD11b+ cells (FIG. 13, MRC-5+4TF). Furthermore, the expression of HLA-DR and CD11c was increased in the CD45+CD11b+ cells compared to the CD45-CD11b- cells (FIGS. 14 and 15). Similarly, CD45+CD11b+HLA-DR+CD11c+ cells were also observed when the human immortalized AD-MSC line SCRC-4000 (ATCC) was used instead of MRC-5 (data not shown). These results indicate that icDC2 can be produced from a human fibroblast or fibroblast-like cell by the four transcription factors, human PU. 1, KLF4, IRF4 and C/EBP alpha.

On the other hand, when the same analysis was performed using the human lung epithelial cell line A549 (Riken), the human pancreatic cancer cell line Capan-1 (ATCC), and the human cervical cancer cell line HeLa (ATCC) instead of MRC-5, CD45+CD11b+HLA-DR+CD11c+ cells were not observed (data not shown).

### 8. Induction of acquired immune response by cDC2-like cells transdifferentiated by four transcription factors

The four transcription factors were introduced into MEFs by the procedure disclosed in 1 above, and the antigen-presenting ability of the resulting cDC2-like cells was examined. Spleens were collected from the OT-II mice (Chicken ovalbumin 323-339 epitope-specific T cell receptor transgenic mice), digested in collagenase buffer, and then ground with glass slides to collect splenocytes. CD4+ T Cell Isolation Kit, mouse (Miltenyi Biotec Inc.) was used to isolate CD4-positive T cells from the splenocyte population according to the kit instructions. MEFs, AD-MSCs, or immortalized mouse embryonic fibroblasts prepared by the 3T3 method (Todaro and Green, J. Cell Biol, 1962) (hereinafter referred to as "3T3") introduced with the four transcription factors; MEFs (negative control); or GM-DC (positive control) (2×10⁴ cells each) were mixed with 2×10⁴ CD4-positive T cells, or were not mixed (mock, negative control), and placed in a round-bottom 96-well plate. The CD4-positive T cells were cultured for 7 days with or without the addition of 10 µg/mL of chicken ovalbumin 323-339 peptide. Total cell count, CD3 epsilon-positive cell count, and CD69 expression were observed by FACS Verse (BD Biosciences).

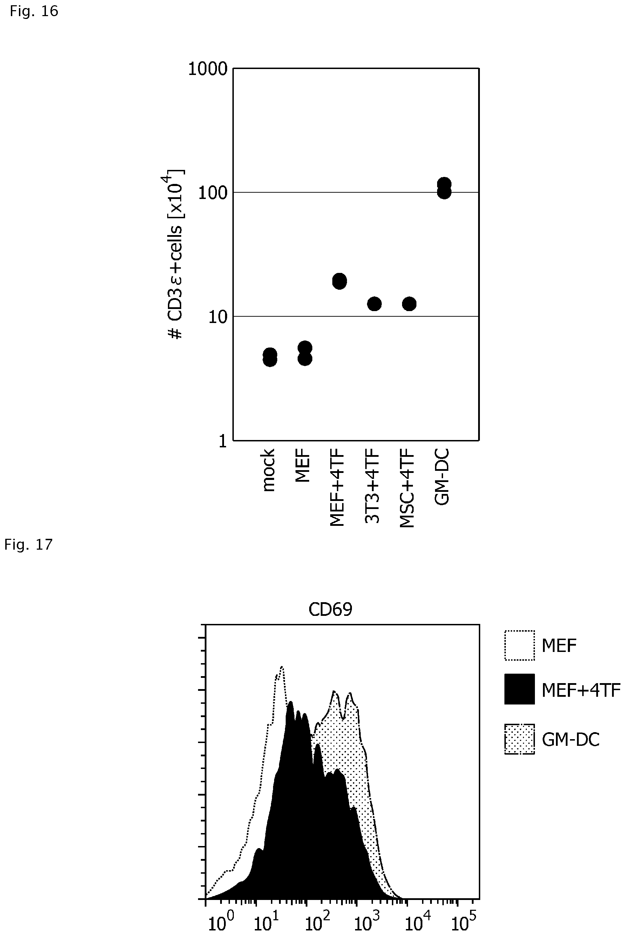

The results are shown in FIGS. 16 and 17. The number of CD3 epsilon-positive cells was increased in the mixed cell population of MEFs introduced with the four transcription factors (MEF + 4TF) and T cells, the mixed cell population of MSCs introduced with the four transcription factors (MSC + 4TF) and T cells, and the mixed cell population of 3T3s introduced with the four transcription factors (3T3 + 4TF) and T cells, confirming that the T cells had proliferated (FIG. 16). The increased expression of the activated T cell marker CD69 was also confirmed in the mixed cell population of MEFs introduced with the four transcription factors (MEF + 4TF) and T cells (FIG. 17). These results indicate that the cDC2-like cells transdifferentiated from MEFs by the four transcription factors have antigen-presenting ability.

### 9. Induction of anti-tumor immune response in cancer mouse model by cDC2-like cells transdifferentiated by four transcription factors

The procedure for evaluating whether cDC2-like cells transdifferentiated by the four transcription factors can be pulsed with cancer antigens and used as a DC vaccine, using a cancer mouse model, is shown below.

### (9-1) Production of tumor-bearing model mice

The B 16F 1 melanoma cells (1×10⁶ cells) were subcutaneously implanted into C57BL/6 mice. Tumor size was measured after one week, and the mice were used as a tumor-bearing model.

### (9-2) Preparation and administration of pulsed cDC2-like cells

The four transcription factors were introduced according to the same procedure as disclosed in 1 above to induce cDC2-like cells from fibroblasts derived from C57BL/6 CD45.1 mice. The cDC2-like cells were loaded with lysate of the B16F1 melanoma cells and were cultured for 16 hours. The cDC2-like cells were then intravenously administered to the above tumor-bearing model mice, and tumor size was measured every week.

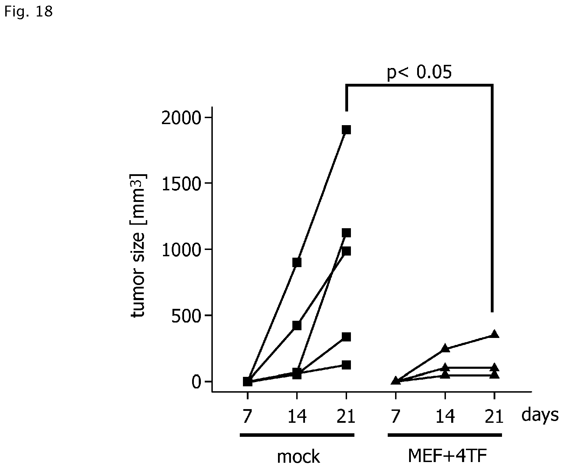

The results are shown in FIG. 18. In the figure, "days" indicates the number of days since the subcutaneous implantation of B16F1 melanoma cells. When the tumor sizes in the group administered with MEFs introduced with the four transcription factors (MEF + 4TF) and in the group that was not administered with anything (mock, negative control) were measured and compared, significant differences were observed at 21 days after subcutaneous implantation (i.e., 14 days after MEF + 4TF administration) (p<0.05, Student's t-test). These results indicate that the cDC2-like cells transdifferentiated from MEFs by the four transcription factors have cancer-shrinking properties.

## Claims

1. A method for producing a conventional type-2 dendritic cell (cDC2)-like cell from a fibroblast or a fibroblast-like cell, the method comprising a step of introducing a nucleic acid encoding PU. 1, a nucleic acid encoding KLF4, a nucleic acid encoding IRF4 and a nucleic acid encoding C/EBP into the fibroblast or the fibroblast-like cell.

2. The method according to claim 1, wherein the C/EBP is C/EBP alpha or C/EBP beta.

3. The method according to claim 1 or 2, wherein the fibroblast or fibroblast-like cell is a cancer-associated fibroblast.

4. The method according to claim 1 or 2, wherein the fibroblast or fibroblast-like cell is a mesenchymal stem cell.

5. A pharmaceutical composition for preventing or treating cancer in a subject, the pharmaceutical composition comprising cDC2-like cells prepared by the method according to any one of claims 1 to 4.

6. The pharmaceutical composition according to claim 5, wherein the cDC2-like cell is loaded with a cancer antigen.

7. The pharmaceutical composition according to claim 5 or 6, wherein the cDC2-like cell is autologous to the subject.

8. The pharmaceutical composition according to any one of claims 5 to 7, wherein the cDC2-like cell is immortalized.

9. A composition for transdifferentiating a fibroblast or a fibroblast-like cell into a conventional type-2 dendritic cell-like cell, the composition comprising a nucleic acid encoding PU.1, a nucleic acid encoding KLF4, a nucleic acid encoding IRF4 and a nucleic acid encoding C/EBP.

10. The composition according to claim 9, wherein the C/EBP is C/EBP alpha or C/EBP beta.
